Europäisches Patentamt

European Patent Office

Office européen des brevets

(19)

(11) Veröffentlichungsnummer: **0 108 820**
**B1**

# (12) EUROPÄISCHE PATENTSCHRIFT

(45) Veröffentlichungstag der Patentschrift:
**08.10.86**

(21) Anmeldenummer: **82110374.4**

(22) Anmeldetag: **10.11.82**

(51) Int. Cl.⁴: **A 61 K 9/00**

(54) Infusionslösungen zur Herztherapie.

(43) Veröffentlichungstag der Anmeldung:
**23.05.84 Patentblatt 84/21**

(45) Bekanntmachung des Hinweises auf die Patenterteilung:
**08.10.86 Patentblatt 86/41**

(84) Benannte Vertragsstaaten:
**AT BE CH DE FR GB IT LI LU NL SE**

(56) Entgegenhaltungen:
**AU - B - 3 793**

**CHEMICAL ABSTRACTS, Band 89, Nr. 21, 20. November 1978, Seite 63, Nr. 173760p, Columbus, Ohio, USA, W. RUDOWSKI et al.: "Infusion of physiological saline supplemented with adenine, pyruvate and phosphate"**
**CHEMICAL ABSTRACTS, Band 72, Nr. 1, 5. Januar 1970, Seite 139, Nr. 10633j, Columbus, Ohio, USA, MONROE S. KARETZKY et al.: "Effect of sodium pyruvate infusion on acid-base balance and gas exchange in the dog"**

(73) Patentinhaber: **Laevosan-Gesellschaft m.b.H., Estermannstrasse 17, A-4020 Linz (AT)**

(72) Erfinder: **Gerlach, Eckehart, Prof., Irschenhauserstrasse 17, D-8021 Ecking (DE)**

(74) Vertreter: **Huber, Bernhard, Dipl.-Chem. et al, Patentanwälte H. Weickmann, Dr. K. Fincke F.A. Weickmann, B. Huber Dr. H. Liska, Dr. J. Prechtel Möhistrasse 22 Postfach 860 820, D-8000 München 86 (DE)**

# Beschreibung

Die Erfindung betrifft Infusionslösungen zur Herztherapie, wie z.B. zur Behandlung der Herzinsuffizienz oder von Myokardschäden.

In der heutigen, von Leistungsdruck und Stress und damit verbunden oft auch von falscher Ernährung und Bewegungsarmut geprägten Zeit treten die Erkrankungen des Herzens und des Kreislaufsystems mehr und mehr als Todesursache in den Vordergrund. Als häufigste und schwerwiegendste Folgeerscheinung tritt dabei die Herzinsuffizienz auf, d.h. das Herz ist (belastungs-)insuffizient geworden.

Unter den Mitteln zur Behandlung von Herzkrankheiten, insbesondere der Herzinsuffizienz, nehmen die Herzglykoside eine bevorzugte Stellung ein. Die Herzglykoside steigern die Kontraktionskraft des Herzmuskels (positive inotrope Wirkung), besitzen aber den Nachteil, dass sie die Reizbildung am Herzen fördern (positive bathmotrope Wirkung). Weitere bevorzugt angewandte Verbindungen mit kontraktilitätssteigernder Wirkung sind die Sympathicomimetica (z.B. die Katecholamine Adrenalin, Dopamin, Dobutamin und Noradrenalin), die eine ausgeprägte positive inotrope Wirkung besitzen.

Diese Wirkung ist allerdings nur von relativ kurzer Dauer und daher für die Therapie der chronischen Herzinsuffizienz nicht geeignet. Trotz vieler Bemühungen zeichnete sich bisher keine Entwicklung für ein neues therapeutisches Prinzip in der Therapie der Herzinsuffizienz ab.

Aufgabe der vorliegenden Erfindung ist deshalb die Bereitstellung weiterer Mittel zur Behandlung von Herzkrankheiten, die die Nachteile der bisher auf diesem Gebiet bekannten Mittel vermeiden. Diese Aufgabe wird mit der vorliegenden Erfindung gelöst.

Gegenstand der vorliegenden Erfindung sind Infusionslösungen zur Herztherapie, die dadurch gekennzeichnet sind, dass sie Brenztraubensäure und/oder deren physiologisch verträglichen Salze und ein oder mehrere C5-Zucker enthalten.

Aus Chemical Abstracts, Band 89, Nr. 173760p (1978) war es bekannt, dass die Infusion von mit Adenin, Pyruvat und Phosphat ergänzter physiologischer Kochsalzlösung den 2,3-Diphosphoglyceratspiegel und den Partialdruck, bei dem Hämoglobin zu 50% Sauerstoff gesättigt ist, anhebt. Aus Chemical Abstracts, Band 72, Nr. 10633j (1970) war es ausserdem bekannt, dass die Infusion von Natriumpyruvat nach vollständiger β-adrenergischer Blockade mit Propranolol eine deutliche Steigerung der Sauerstoffaufnahme hervorruft. Daraus konnte jedoch nicht geschlossen werden, dass eine Infusionslösung, welche Brenztraubensäure in Kombination mit C5-Zucker enthält, eine besondere Eignung für die Herztherapie aufweist. Ausserdem war es aus der AU-P-3793 bekannt, zur Behandlung von Kreislaufschwierigkeiten Glycerin in Kombination mit Bernsteinsäure, Brenztraubensäure und Fumarsäure zu verabreichen, wobei dieser Kombination eine emulgierende Wirksamkeit zugeschrieben wurde. Erwähnt ist jedoch nur eine orale Verabreichung. Ein Hinweis auf eine Infusionslösung gemäss der Erfindung lässt sich daraus nicht entnehmen.

Als Salze der Brenztraubensäure können alle Salze mit physiologisch verträglichen Kationen, die in der Infusionslösung ausreichend löslich sind, verwendet werden. Vorzugsweise sind dies das Kalium-, Calcium-, Magnesium- und/oder Ammoniumsalz, und insbesondere das Natriumsalz. Es können auch Mischungen von Brenztraubensäure mit einem oder mehreren ihrer Salze verwendet werden. Die Gesamtmenge an Brenztraubensäure und/oder ihren Salzen beträgt vorzugsweise zwischen 0,5 bis 15, insbesondere zwischen 2 und 10 Gew.-%, bezogen auf die gesamte Infusionslösung. Bevorzugte Ausführungsformen sind z.B. Infusionslösungen mit einem Gehalt an 2,5, 5 und 10 Gew.-% Natriumpyruvat.

Unter C5-Zucker werden die eigentlichen Zucker (Pentosen) wie Ribose, Arabinose, Xylose und Lyxose, und die davon abgeleiteten Zuckeralkohole (Pentitol) verstanden. Es können ein oder mehrere Zucker und/oder Zuckeralkohole zusammen mit der Brenztraubensäure und/oder deren Salzen eingesetzt werden. Bevorzugte C5-Zucker sind Xylose und der entsprechende Zuckeralkohol. Die Gesamtmenge an C5-Zucker beträgt vorzugsweise 0,2 bis 5, insbesondere 1 bis 3 Gew.-%, bezogen auf die fertige Infusionslösung.

Die erfindungsgemässen Infusionslösungen enthalten die Brenztraubensäure und/oder deren Salze und den C5-Zucker in einem für Infusionszwecke üblichen Medium, wie z.B. in physiologischen Salzlösungen, Plasmasalzlösungen und/oder nährstoffhaltigen Lösungen, wobei auch Gemische dieser Lösungen verwendet werden können. Die fertigen Infusionslösungen sollen den Anforderungen der Arzneibücher am Ort ihrer Verwendung entsprechen (z.B. Deutsches Arzneibuch, Europäisches Arzneibuch, Österreichisches Arzneibuch usw.). Sie sollen insbesondere Blutisotonie und Blutisoionie und den pH-Wert des Blutes aufweisen, und ausserdem nur destilliertes oder demineralisiertes Wasser enthalten und pyrogenfrei sein. Zur Angleichung an den pH-Wert des Blutserums (7,36 bis 7,42) und zu dessen Konstanthaltung kann es, insbesondere bei Verwendung der freien Brenztraubensäure, erforderlich sein, den Infusionslösungen verträgliche Puffersubstanzen, wie z.B. Bicarbonat- oder Phosphat-Puffer, zuzusetzen.

Für Infusionszwecke übliche Nährlösungen können enthalten: Kohlenhydrate, wie z.B. Zucker, z.B. Glucose, oder Zuckeralkohole, wie z.B. Sorbitol; Äthanol; essentielle und semiessentielle Aminosäuren; Vitamine; Fette, wie z.B. Pflanzenöle oder synthetische gesättigte Glyceride; Emulgatoren, wie z.B. Phosphatide, insbesondere Lecithin; und Stabilisatoren, wie z.B. Tokopherole. Diese Bestandteile können einzeln oder auch im Gemisch verwendet werden, wobei die Gesamtkonzentration in der Regel 2 bis 15, vorzugsweise 5 bis 10 Gew.-%, bezogen auf die gesamte Infusionslösung, beträgt.

Die Infusionslösungen können auch wässrige Lösungen von organischen hydrophilen, nichttoxischen Colloiden, wie z.B. Oxypolygelatine, Dextran und/oder Hydroxyäthylstärke, enthalten.

Gegenstand der Erfindung ist auch ein Verfahren zur Herstellung der erfindungsgemässen Infusionslösungen, das dadurch gekennzeichnet ist, dass man Brenztraubensäure und/oder deren physiologisch verträgliche Salze und ein oder mehrere C5-Zucker, vorzugsweise in lyophilisierter Form, in einem für Infusionszwecke üblichen Medium, wie z.B. in physiologischen Salzlösungen, Plasmasalzlösungen und/oder nährstoffhaltigen Lösungen auflöst. Die Brenztraubensäure und deren Salze werden dabei vorzugsweise in lyophilisierter Form eingesetzt. Gleiches gilt für die C5-Zucker. Zur Sicherstellung, dass die Infusionslösung keine ungelösten (suspendierten) Bestandteile enthält, kann gegebenenfalls auch eine Filtration, vorzugsweise unter Inertgasdruck, erforderlich sein. Die Infusionslösung wird dann in geeignete Infusionsflaschen abgefüllt. Die Abfüllung geschieht zweckmässigerweise mittels Sterilfiltration; es kann aber auch, gegebenenfalls zusätzlich, eine Sterilisation der fertig abgefüllten Infusionsbehälter erfolgen. In einer Verfahrensvariante werden die Brenztraubensäure und/oder der Salze und C5-Zucker, in fester Form in die Infusionsbehälter abgefüllt und erst kurz vor Gebrauch im Infusionsmedium verbrauchsfertig aufgelöst. Dieses Verfahren hat sich in verschiedener Hinsicht (Lagerung, Transport, Stabilität der Lösungen, Wahl des Infusionsmediums, je nach Zweckbestimmung) als sehr zweckmässig erwiesen. Die Verfahrensbedingungen (Lösungs- und Mischungsbedingungen, Filtration, Abfüllung) werden dabei so gewählt, dass die zum Gebrauch bestimmten Infusionslösungen den Anforderungen an die Sterilität und Pyrogenfreiheit erfüllen. Einzelne oder alle Verfahrensschritte werden daher zweckmässig unter Inertgas, insbesondere unter Stickstoff oder Kohlendioxid, durchgeführt. Zur Sicherstellung der Stabilität der Infusionslösungen über einen längeren Aufbewahrungszeitraum kann es auch vorteilhaft sein, eingefärbte Infusionsbehälter zu verwenden und/oder geeignete Stabilisatoren, wie z.B. Tokopherole, zuzusetzen.

Die erfindungsgemässen Infusionslösungen eignen sich sehr gut für die Behandlung von Herzkrankheiten, wie: energetische Herzinsuffizienz verschiedener Genese (wie Mangel an energiereichen Phosphaten, mangelhafter Transfer von Reduktionsäquivalenten vom Cytosol in das mitochondriale Kompartiment, mangelhafte Verwertung von Substraten, die zur Aufrechterhaltung der Herzleistung dienen), Myokardschäden (Hypoxie), zur Verkleinerung der ischämischen Randzone nach einem Myokardinfarkt während der ersten 36 Stunden, bei chirurgischen Eingriffen am Herzen (insbesondere bei Bypass-Patienten, Klappenersatz und Septumdefekten) sowie bei Stoffwechselstörungen.

Die erzielten Ergebnisse sind im Prinzip mit jenen der Katecholamine zu vergleichen; gegenüber den Katecholaminen besitzen die erfindungsgemässen Infusionslösungen aber den Vorteil, dass sie sich auch dort noch anwenden lassen, wo Katecholamine bereits versagen.

Patienten, die eine energetische Herzinsuffizienz haben, die durch Katecholamin-Zufuhr nicht mehr behoben werden kann, wurde in einer Dosierung von 0,5 g/kg/h Natriumpyruvat infundiert. Unter dieser Infusion kam es zu einem systolischen Druckanstieg von 20 mm Hg bei gleichbleibendem peripherem Widerstand und bei gleichbleibender Herzfrequenz.

Die Verträglichkeit der pyruvathaltigen Infusionslösungen ist ausgezeichnet. In einer pharmakokinetischen Studie, in der gesunden Versuchspersonen Infusionslösungen mit unterschiedlichen Konzentrationen (2,5, 5 und 10 Gew.-%) Natriumpyruvat verabreicht wurden (45 oder 90 min Dauerinfusion) wurden alle Konzentrationen von allen Versuchspersonen sehr gut vertragen. Der Pyruvatspiegel war während der gesamten Infusionsdauer gleichbleibend erhöht.

Besonders gut bewährt haben sich die erfindungsgemässen Infusionslösungen auch im Falle des Herzversagens während Herzoperationen, wenn die Infusion sofort während der Operation beginnt.

Die Infusion kann intraarteriell, insbesondere intracoronariell, oder intravenös erfolgen. Die Dosierung (Konzentration) und die Infusionsdauer richten sich im allgemeinen nach der Art und Schwere der Erkrankung, aber auch nach dem Allgemeinbefinden des Patienten.

Das nachfolgende Beispiel zeigt die Herstellung einer pyruvathaltigen Infusionslösung.

Beispiel

Herstellung einer Infusionslösung

| Bestandteile | Gew.-Teile |
|---|---|
| Natriumpyruvat | 50,0 |
| Ribose | 10,0 |
| Natriumchlorid | 6,0 |
| Kaliumchlorid | 0,4 |
| Calciumchlorid (Krist.) | 0,4 |
| destilliertes Wasser | ad 1000 |

werden unter Stickstoffatmosphäre gelöst und sterilisiert, wobei das Filtrat direkt in eine Infusionsflasche abgefüllt wird.

**Patentansprüche**

1. Infusionslösung zur Herztherapie, dadurch gekennzeichnet, dass sie Brenztraubensäure und/oder deren physiologisch verträglichen Salze und ein oder mehrere C5-Zucker enthält.

2. Infusionslösung nach Anspruch 1, dadurch gekennzeichnet, dass sie das Natrium-, Kalium-, Calcium-, Magnesium- und/oder Ammoniumsalz der Brenztraubensäure enthält.

3. Infusionslösung nach einem der vorhergehenden Ansprüche, dadurch gekennzeichnet, dass sie das Natriumsalz der Brenztraubensäure enthält.

4. Infusionslösung nach einem der vorhergehenden Ansprüche, dadurch gekennzeichnet, dass sie die Brenztraubensäure und/oder deren Salze in einer Menge von 0,5 bis 15, insbesondere von 2 bis 10 Gew.-%, bezogen auf die gesamte Infusionslösung, enthält.

5. Infusionslösung nach einem der vorhergehenden Ansprüche, dadurch gekennzeichnet, dass sie wenigstens eine Pentose und/oder einen Pentit enthält.

6. Infusionslösung nach einem der vorhergehenden Ansprüche, dadurch gekennzeichnet, dass sie 0,2 bis 5, insbesondere 1 bis 3 Gew.-% C5-Zucker enthält.

7. Infusionslösung nach einem der vorhergehenden Ansprüche, dadurch gekennzeichnet, dass sie die Brenztraubensäure oder deren Salze und die C5-Zucker in einer für Infusionszwecke üblichen physiologischen Salzlösung, Plasmasalzlösung und/oder nährstoffhaltigen Lösung, gegebenenfalls zusammen mit physiologisch verträglichen Puffersubstanzen, enthält.

8. Verfahren zur Herstellung einer Infusionslösung nach einem der Ansprüche 1 bis 7, dadurch gekennzeichnet, dass man Brenztraubensäure und/oder deren physiologisch verträglichen Salze und C5-Zucker in einem für Infusionszwecke üblichen Medium, gegebenenfalls zusammen mit physiologisch verträglichen Puffersubstanzen, löst.

9. Verfahren nach Anspruch 8, dadurch gekennzeichnet, dass man als Medium eine physiologische Salzlösung, Plasmasalzlösung und/oder nährstoffhaltige Lösung verwendet.

10. Verfahren nach Anspruch 8 oder 9, dadurch gekennzeichnet, dass man die Brenztraubensäure und/oder deren Salze in lyophilisierter Form einsetzt.

11. Verfahren nach einem der Ansprüche 8 bis 10, dadurch gekennzeichnet, dass man Brenztraubensäure und/oder deren Salze und C5-Zucker in fester Form in Infusionsbehälter bringt und kurz vor ihrer Verwendung in einem für Infusionszwecke üblichen Medium auflöst.

## Claims

1. Infusion solution for heart therapy, characterised in that it contains pyruvic acid and/or its physiologically acceptable salts and one or more C5-sugars.

2. Infusion solution according to claim 1, characterised in that it contains the sodium, potassium, calcium, magnesium and/or ammonium salt of pyruvic acid.

3. Infusion solution according to one of the preceding claims, characterised in that it contains the sodium salt of pyruvic acid.

4. Infusion solution according to one of the preceding claims, characterised in that it contains pyruvic acid and/or its salts in an amount of 0.5 to 15, especially from 2 to 10 wt.-%, referred to the whole infusion solution.

5. Infusion solution according to one of the preceding claims, characterised in that it contains at least one pentose and/or pentitol.

6. Infusion solution according to one of the preceding claims, characterised in that it contains 0.2 to 5, especially 1 to 3 wt.-% of C5-sugar.

7. Infusion solution according to one of the preceding claims, characterised in that it contains pyruvic acid or its salts and the C5-sugar in a physiological salt solution usual for infusion purposes, plasma salt solution and/or nutrient-containing solution, possibly together with physiologically acceptable buffer substances.

8. Process for the production of an infusion solution according to one of claims 1 to 7, characterised in that one dissolves pyruvic acid and/or its physiologically acceptable salts and C5-sugar in a medium usual for infusion purposes, possibly together with physiologically acceptable buffer substances.

9. Process according to claim 8, characterised in that, as medium, one uses a physiological salt solution, plasma salt solution and/or nutrient-containing solution.

10. Process according to claim 8 or 9, characterised in that one uses the pyruvic acid and/or its salts in lyophilised form.

11. Process according to one of claims 8 to 10, characterised in that one introduces pyruvic acid and/or its salts and C5-sugar in solid form into infusion containers and, shortly before their use, dissolves in a medium usual for infusion purposes.

## Revendications

1. Solution d'infusion pour le traitement du coeur, caractérisé en ce qu'elle contient de l'acide pyruvique et/ou ses sels supportables physiologiquement et un ou plusieurs sucres en C5.

2. Solution d'infusion selon la revendication 1, caractérisé en ce qu'elle contient le sel de sodium, de potassium, de calcium, de magnésium et/ou d'ammonium de l'acide pyruvique.

3. Solution d'infusion selon l'une des revendications précédentes, caractérisée en ce qu'elle contient le sel de sodium de l'acide pyruvique.

4. Solution d'infusion selon l'une des revendications précédentes, caractérisée en ce qu'elle contient l'acide pyruvique et/ou ses sels dans une quantité de 0,5 à 15, en particulier de 2 à 10% en poids, rapportés à la solution d'infusion totale.

5. Solution d'infusion selon l'une des revendications précédentes, caractérisée en ce qu'elle contient au minimum un pentose et/ou un pentitol.

6. Solution d'infusion selon l'une des revendications précédentes, caractérisée en ce qu'elle contient de 0,2 à 5, en particulier de 1 à 3% en poids de sucre en C5.

7. Solution d'infusion selon l'une des revendications précédentes, caractérisée en ce qu'elle contient l'acide pyruvique ou ses sels et les sucres en C5 dans une solution saline physiologique dans une solution saline plasmatique et/ou dans une solution contenant des substances nutritives

**0108820**

usuelles pour les infusions, le cas échéant conjointement avec des substances tampons physiologiquement supportables.

8. Procédé pour la préparation d'une solution d'infusion selon l'un des revendications 1 à 7, caractérisé en ce que l'on dissout de l'acide pyruvique et/ou ses sels physiologiquement supportables et les sucres en C5 dans un milieu usuel pour infusions, le cas échéant conjointement avec des substances tampons physiologiquement supportables.

9. Procédé selon la revendication 8, caractérisé en ce que l'on utilise en tant que milieu une solution saline physiologique, une solution saline plasmatique et/ou une solution contenant des substances nutritives.

10. Procédé selon la revendication 8 ou 9, caractérisé en ce que l'on met en oeuvre l'acide pyruvique et/ou ses sels sous forme lyophilisée.

11. Procédé selon l'une des revendications 8 à 10, caractérisé en ce que l'on introduit de l'acide pyruvique et/ou ses sels et des sucres en C5 sous forme solide dans un récipient d'infusion et en ce qu'on les dissout peu de temps avant leur utilisation dans un milieu usuel pour infusions.